# EUROPEAN PATENT SPECIFICATION

(11) **EP 2 059 203 B1**
(45) Date of publication and mention of the grant of the patent: **01.02.2017**
(21) Application number: 07814263.5
(22) Date of filing: 20.08.2007
(51) Int. Cl.: A61F 9/008, A61F 2/14, A61F 9/009, A61F 9/013

(54) **SYSTEM FOR RESECTING CORNEAL TISSUE**
SYSTEM ZUR RESEKTION VON HORNHAUTGEWEBE
SYSTÈME DE RÉSECTION DU TISSU CORNÉEN

(30) Priority: 05.09.2006 US 469899
(43) Date of publication of application: 20.05.2009
(73) Proprietor: AMO Development, LLC, Santa Ana, CA 92705 (US)
(72) Inventor: KURTZ, Ronald, M., Irvine, CA 92603 (US); JUHASZ, Tibor, Irvine, CA 92620 (US); SARAYBA, Melvin, A., Ladera Ranch, CA 92694 (US); STEINERT, Roger, Laguna Beach, CA 92651 (US)
(74) Representative: Clark, Jane Anne
(86) International application number: PCT/US2007/076335
(87) International publication number: WO 2008/030699

(56) References cited:
- WO-A1-94/09849
- WO-A1-94/25107
- WO-A1-2004/017878
- DE-C1- 10 124 358
- US-A- 4 772 283
- US-A- 5 549 632
- US-A- 6 110 166
- US-B1- 6 325 792

## Description

### BACKGROUND OF THE INVENTION

The field of the present invention is systems and procedures for transplanting corneas.

A variety of techniques presently exist for performing corneal transplants. The tools used for the different techniques range from the traditional trephine to the more advanced laser surgical systems. Regardless of the technique or tool used for the transplant procedure, the overarching goal remains to provide the recipient with new corneal tissue while minimizing post-surgical complications such as induced astigmatism. Other desirable aspects of recent improvements include reducing the overall healing time and the likelihood of additional complications from wound ruptures.

One technique which attempts to use advanced laser surgical systems to help reduce post-surgical complications is disclosed in U.S. Patent No. 6,805,694. This technique relies on the theory that the inner and outer diameters of the undercut region should be chosen to have a specific ratio. Further, the theory postulates that the internal pressure of the eye will provide enhanced wound stability and reduce post-surgical complications by selection of an appropriate ratio. Unfortunately, when applied in actual transplant procedures, this theory does not rise up to meet expectations.

US 2006/0100612 describes a laser-based device for non-mechanical three-dimensional trepanation during cornea transplants. As shown in Figures 2 and 3 the donor cornea has a saw-toothed shaped raised areas along its rim whilst as shown in Figures 4 and 5 the donor cornea has a projection and as shown in Figures 6 and 7 the donor cornea has a radial denticulation for radial grooves, with in each case the recipient cornea having complementary features.

US 2002/0173779 describes a laser system for corneal grafting in which according to a preferred configuration the undercut is zig zag shaped.

### BRIEF SUMMARY OF THE INVENTION

The present invention set out in the appended claims. Described herein are a system and method for resecting corneal tissue as part of a transplant procedure. In the system, a surgical laser emits a pulsed laser beam which is directed into the cornea by a focusing assembly. An interface provides a plurality of incision patterns for selection of a sidecut pattern which includes an annular region. The selected sidecut pattern is received by a controller which employs the focusing assembly to move the focal point of the pulsed laser beam and incise a donor cornea according to the sidecut pattern, placing the incision corresponding to the annular region at a predetermined depth from the anterior corneal surface. The controller also employs the focusing assembly to move the focal point and incise a recipient cornea according to the sidecut pattern, again placing the incision corresponding to the annular region at the same predetermined depth from the anterior corneal surface.

In the method, the depth of an incision from the anterior corneal surface is initially determined. This incision depth is used for both the recipient cornea and the donor cornea, and in each cornea, an annular incision is made at the incision depth. A first sidecut incision is made running from the outer periphery of the annular incision toward one of the anterior corneal surface or the posterior corneal surface. A second sidecut incision is made running from the inner periphery of the annular incision toward the other of the anterior corneal surface or the posterior corneal surface. The angle of each sidecut incision to the annular incision may be acute, perpendicular, or obtuse. The combination of the incisions in each cornea resects corneal tissue from the recipient cornea and donor tissue from the donor cornea, respectively. The donor tissue is thereafter grafted into the recipient cornea. Following the graft, the donor tissue may be secured using a plurality of sutures, each preferably passing through the sidecut incision which is nearest the posterior surface of the recipient cornea.

The extent of the sidecut incisions depends upon whether the transplant is a full thickness corneal transplant or a partial thickness corneal transplant. In a full thickness transplant, each sidecut incision runs from the annular incision to one of the anterior corneal surface or the posterior corneal surface. A partial thickness transplant may be an anterior lamellar keratoplasty (ALK) or a posterior lamellar keratoplasty (PLK). In both procedures, a resection incision is made within the stromal tissue of each cornea. For the ALK procedure, the depth of the annular incision is between the anterior surface and the resection incision, whereas for the PLK procedure, the depth of the annular incision is between the posterior surface and the resection incision. Thus, in the ALK procedure, one of the sidecut incisions runs from the annular incision to the anterior surface and the other runs from the annular incision to the resection incision. Similarly, in the PLK procedure, one of the sidecut incisions runs from the annular incision to the posterior surface and the other runs from the annular incision to the resection incision.

Accordingly, an improved system and method for resecting corneal tissue as part of a transplant procedure are disclosed. Advantages of the improvements will appear from the drawings and the description of the preferred embodiment.

### BRIEF DESCRIPTION OF THE DRAWINGS

In the drawings, wherein like reference numerals refer to similar components:

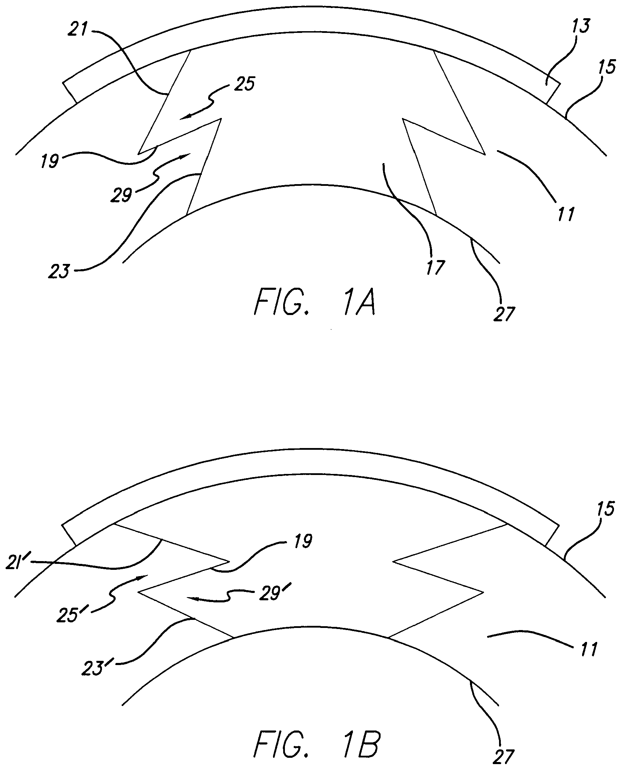

Fig. 1A & 1B illustrates a sectional view of a cornea with incisions made therein for a full thickness corneal tissue transplant;

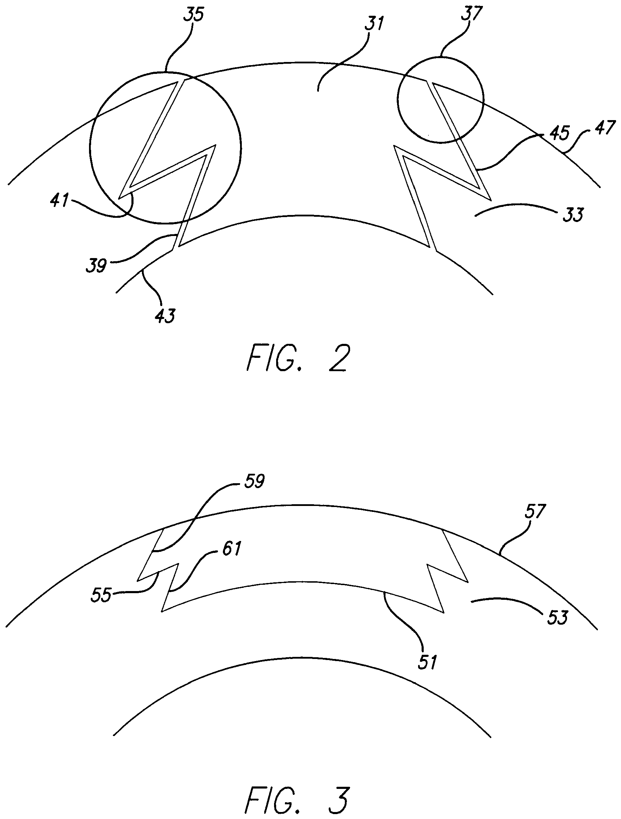

Fig. 2 illustrates a sectional view of donor tissue grafted into a recipient cornea;
Fig. 3 illustrates a sectional view of incisions made in a cornea for a partial thickness ALK corneal tissue transplant;

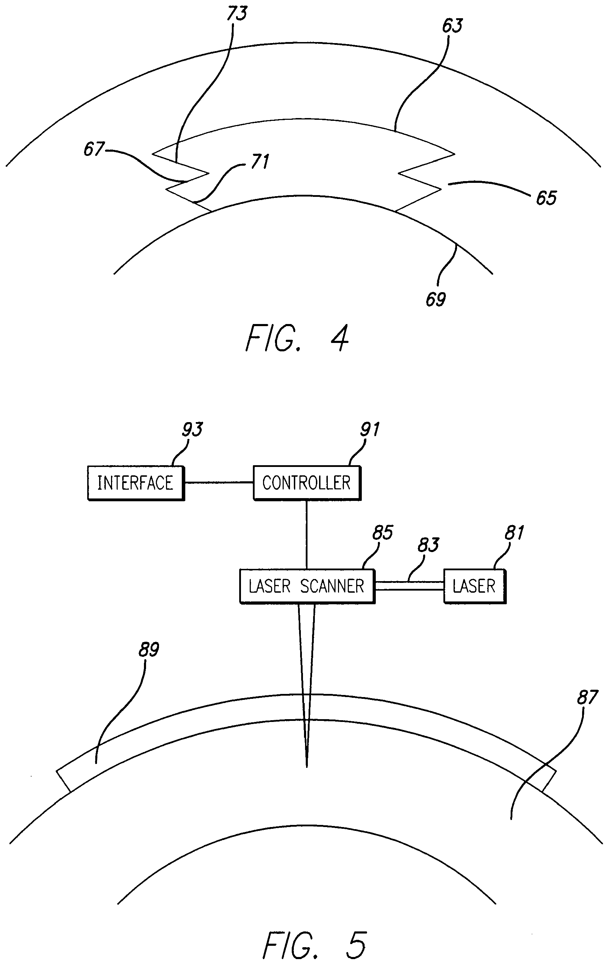

Fig. 4 illustrates a sectional view of incisions made in a cornea for a partial thickness PLK corneal tissue transplant; and
Fig. 5 is a schematic view of a system for resecting corneal tissue.

### DETAILED DESCRIPTION OF THE INVENTION

Turning in detail to the drawings, Fig. 1A illustrates the incisions made in a cornea 11 as part of a full thickness corneal transplant procedure. The same incisions are made in both the recipient cornea and the donor cornea, although the incisions in the donor cornea may be made approximately 1%-5% larger, or more preferably 2%-5% larger, than the incisions in the recipient cornea to account for shrinkage in the donor corneal tissue following resection. As part of the procedure, a contact lens 13 placed against the anterior corneal surface 15. This contact lens 13 deforms the cornea 11, forcing the anterior corneal surface 15 to take on the shape of the contact lens 13. Deformation of the cornea 11 in this manner provides multiple advantages which are well known to skilled artisans. For example, U.S. Patent No. 5,549,632 describes advantages gained in making laser incisions by deforming the shape of the cornea, particularly by applanation. U.S. Patent No. 6,863,667 and U.S. Pat. App. No. 11/258,399 describe patient interface devices which may be used to align the surgical laser with the recipient cornea for purposes of making accurate incisions.

Further, deformation of the cornea reduces the amount of physical data which needs to be collected for both the recipient cornea and the donor cornea prior to the transplant procedure. The physical data collected includes thickness measurements of both the recipient and donor corneas. These thickness measurements are used to develop a thickness profile of each cornea. Additional physical data may also be collected for each cornea. This thickness profile, along with any other data needed for the procedure, may be obtained by any one of the many known methods for measuring the physical structure of the eye, with the preferred method being through optical coherence tomography (OCT). Many commercially available OCT scanners are capable of performing such measurements. One example is the VisanteTM OCT scanning system, manufactured by Carl Zeiss Meditec, which has an office in Dublin, California. One advantage of the VisanteTM OCT system is that it does not make contact with the cornea when performing the OCT scan.

Three incisions, an annular incision 19 and two sidecut incisions 21, 23, are made in the cornea 11 to resect the corneal tissue 17. These incisions may be made separately, one at a time, or they may be made concurrently. The combined incisions result in corneal tissue being resected from the recipient cornea, and donor tissue being resected from the donor cornea. All incisions are preferably made using a pulsed laser beam having ultra-short pulses, preferably in the femtosecond range. The laser may be of the type described in U.S. Patent No. 4,764,930, producing an ultra-short pulsed beam as described in one or both of U.S. Patent No. 5,984,916 and U.S. Patent No. RE37,585. Commercial lasers capable of performing the incisions are available from IntraLase Corp. of Irvine, California.

The annular incision 19 is located at a predetermined depth from the anterior corneal surface 15. To facilitate the resection and grafting process, the entire annular incision 19 is at a uniform distance from the anterior corneal surface 15, although such uniformity of depth is not required. Further, for simplicity the annular incision 19 is described as being defined by an inner radius and an outer radius, although such is not necessary. It is sufficient for this incision to be formed by an inner perimeter and an outer perimeter, both perimeters being of any desired shape. More complex shapes, however, can add significantly to the complexity of the procedure. Various techniques are known for making the annular incision 19 at a uniform distance from the anterior corneal surface 15. For example, the techniques disclosed in U.S. Patent No. 5,993,438, U.S. Patent No. 6,730,074, and U.S. Patent Publication No. 20050245915 may be readily adapted to make the desired annular incision 19. Other techniques known to skilled artisans may also be employed.

The first sidecut incision 21 runs from the outer periphery of the annular incision 19 to the anterior corneal surface 15. An acute angle is formed at the juncture 25 of the first sidecut incision 21 and the annular incision 19. The second sidecut incision 23 runs from the inner periphery of the annular incision 19 to the posterior corneal surface 27. An acute angle is also formed at the juncture 29 of the second sidecut incision 23 and the annular incision 19.

Fig. 1B illustrates an alternative configuration for the sidecut incisions 21', 23' made in the cornea 11 with respect to the annular incision 19. Here, the first sidecut incision 21' runs from the inner perimeter of the annular incision 19 to the anterior corneal surface 15, and the second sidecut incision 23' runs from the outer perimeter of the annular incision 19 to the posterior corneal surface 27.

The donor tissue 31 is shown grafted into the recipient cornea 33 in Fig 2. Sutures 35, 37 are placed to secure the donor tissue 31 to the recipient cornea 33. Two different techniques for placement of sutures are shown. The first suture 35 is placed through the sidecut incision 39 which runs between the annular incision 41 and the posterior corneal surface 43. The second suture 37 is placed through the sidecut incision 45 which runs between the annular incision 41 and the anterior corneal surface 47. Both locations of sutures are believed to be equally as effective for securing the donor tissue 31 to the recipient cornea 33. Further, neither location is believed to be more likely than the other to induce astigmatism during the healing process.

Fig. 3 illustrates how the incision configuration described above may be adapted for an ALK procedure. For the ALK process, a resection incision 51 is made in the cornea 53 at a predetermined resection depth, and the annular incision 55 is made at a depth which lies between the resection depth and the anterior corneal surface 57. The first sidecut incision 59 is made in the same manner as is described above. The second sidecut incision 61, however, runs from the annular incision to the resection incision 51. The donor tissue may be secured to the recipient cornea by either of the techniques described above.

Fig. 4 illustrates how the incision configuration described above may be adapted for an PLK procedure. For the PLK process, a resection incision 63 is made in the cornea 65 at a predetermined resection depth, and the annular incision 67 is made at a depth which lies between the resection depth and the posterior corneal surface 69. The first sidecut incision 71 is made in the same manner as is described above. The second sidecut incision 73, however, runs from the annular incision to the resection incision 63. In a PLK procedure, sutures are not necessary to secure the donor tissue to the recipient cornea.

Referring to Fig. 5, a surgical system is shown which may be used to incise both a donor cornea or a recipient cornea. Alternatively, two similarly configured systems may be used to incise each respective cornea. A femtosecond surgical laser 81 generates a pulsed laser beam 83 and directs that beam into the focusing assembly 85, which in turn focuses the pulsed beam 83 into the cornea 87. A contact lens 89 is placed over the cornea to deform the anterior corneal surface as described above. Where different contact lenses are used with the donor cornea and the recipient cornea, the posterior curvature, i.e., the side of the contact lens that is placed against the anterior corneal surface, is the same for each contact lens. The controller 91 is a programmable computer which precisely controls the location of the beam focal point within the cornea 87 according to parameters received from the surgeon interface 93. The interface 93 presents the surgeon with several incision patterns from which the desired sidecut pattern is selected. The selected pattern is received by the controller 91, which uses the pattern to incise both the donor cornea and the recipient cornea, placing the annular region of the selected pattern at a predetermined depth from each respective anterior corneal surface. The contact lenses which are used to deform each of the corneas as part of this procedure preferably have the same curvature on the posterior surface, i.e., the surface placed in contact with the anterior corneal surface.

Thus, a method of transplanting corneal tissue is disclosed. While embodiments of this invention have been shown and described, it will be apparent to those skilled in the art that many more modifications are possible without departing from the inventive concepts herein. The invention, therefore, is not to be restricted except by the following claims.

## Claims

1. A system for resecting donor corneal tissue and recipient corneal tissue prior to transplantation of the donor corneal tissue as a corneal implant, the system comprising:
a surgical laser (81) adapted to emit a pulsed laser beam (83);
a focusing assembly (85) adapted to focus the pulsed laser beam into a cornea;
an interface (93) adapted to provide a plurality of incision patterns for selection of a sidecut pattern which includes an annular region; and
a controller (91) in communication with the interface to receive the sidecut pattern, the controller being adapted to move a focal point of the pulsed laser beam within the cornea using the focusing assembly,
direct the focal point of the pulsed laser beam (83) to incise the annular region (19) in the cornea at a predetermined depth from an anterior corneal surface (15) according to the sidecut pattern,
direct the focal point of the pulsed laser beam to make a first sidecut incision (21) in the cornea according to the sidecut pattern, wherein the first sidecut forms a first acute angle with the annular region (19) and runs from an outer periphery of the annular region (19) toward one of a posterior corneal surface (27) and the anterior corneal surface (15), and
direct the focal point of the pulsed laser beam (83) to make a second sidecut (23) incision in the recipient cornea according to the sidecut pattern, wherein the second sidecut forms a second acute angle with the annular region (19) and runs from an inner periphery of the annular region (19) toward the other of the posterior corneal surface (27) and the anterior corneal surface (15)

2. The system of claim 1 further comprising:
a first contact lens (13) adapted to be placed against a donor anterior corneal surface (15), the first contact lens being adapted to conform the donor anterior corneal surface (15) to a shape of the first contact lens; and
a second contact lens adapted to be placed against a recipient anterior corneal surface, the second contact lens being adapted to conform the recipient anterior corneal surface to a shape of the second contact lens, wherein the first and second contact lenses have identical posterior surface curvatures.

3. The system of claim 2, wherein the first contact lens (13) is arranged to applanate the donor anterior corneal surface.

4. A method of resecting donor corneal tissue for transplanting from a donor cornea (11) to a recipient cornea to provide a corneal transplant in the recipient cornea, the method comprising:
determining an incision depth from an anterior corneal surface (15);
making an annular incision (19) at the incision depth in the donor cornea (11);
making a first sidecut incision (21) in the donor cornea (11), the first sidecut incision forming a first acute angle with the annular incision (19) and running from an outer periphery of the annular incision (19) toward one of the anterior corneal surface (15) and a posterior corneal surface (27); and
making a second sidecut incision (23) in the donor cornea (11), the second sidecut incision forming a second acute angle with the annular incision and running from an inner periphery of the annular incision (19) toward the other of the anterior corneal surface (15) and the posterior corneal surface (27), wherein a combination of the incisions in the cornea resects corneal tissue.

5. The method according to claim 4, wherein the first sidecut runs to the anterior corneal surface and the second sidecut runs to the posterior corneal surface.

6. A method according to claim 4, wherein the method further comprises:
determining another incision depth from an anterior corneal surface (15);
making a resection incision (51) at a first one of the incision depths in the donor cornea (11);
making the annular incision (19) at a second one of the incision depths in the donor cornea (11), wherein the second incision depth is less than the first incision depth;
making the first sidecut incision (21) so that the first sidecut in the donor cornea incision runs from the outer periphery of the annular incision (19) to one of the anterior corneal surface (15) and the resection incision (51), rather than to one of the anterior corneal surface and the posterior corneal surface; and
making the second sidecut incision (23) in the donor cornea so that the second sidecut incision runs from the inner periphery of the annular incision (19) to the other of the anterior corneal surface (15) and the resection incision (51), rather than to the other of the anterior corneal surface and the posterior corneal surface, wherein a combination of the incisions in the cornea resects corneal tissue from the cornea.

7. A method according to claim 4, wherein the method further comprises:
determining another incision depth from the anterior corneal surface (15);
making a resection incision (63) at a first one of the incision depths in the donor (11) cornea;
making the annular incision (19) at a second one of the incision depths in the donor cornea (11), wherein the second incision depth is greater than the first incision depth;
making the first sidecut incision (21) in the donor cornea so that the first sidecut incision runs from the outer periphery of the annular incision (19) to one of a posterior corneal surface (27) and the resection incision (63), rather than to one of the anterior corneal surface and the posterior corneal surface; and
making the second sidecut incision (23) in the donor cornea so that the second sidecut incision runs from the inner periphery of the annular incision (19) to the other of the posterior corneal surface (27) and the resection incision (63), rather than to the other of the anterior corneal surface and the posterior corneal surface, wherein a combination of the incisions resects corneal tissue from the cornea.

## Patentansprüche

1. System zur Resektion von Spender-Hornhautgewebe und Empfänger-Hornhautgewebe vor der Transplantation des Spender-Hornhautgewebes als Hornhautimplantat, wobei das System umfasst:
einen Chirurgielaser (81), der dafür geeignet ist, einen gepulsten Laserstrahl (83) auszusenden;
eine Fokussiereinrichtung (85), die dafür geeignet ist, den gepulsten Laserstrahl in eine Hornhaut zu fokussieren;
eine Schnittstelle (93), die dafür geeignet ist, eine Vielzahl von Schnittführungen für die Auswahl einer seitlichen Schnittführung, die eine ringförmige Region enthält, bereitzustellen; und
eine Steuereinheit (91) in Kommunikation mit der Schnittstelle zum Empfangen der seitlichen Schnittführung, wobei die Steuereinheit dafür geeignet ist, einen Fokus des gepulsten Laserstrahls innerhalb der Hornhaut mittels der Fokussiereinrichtung zu verschieben,
den Fokus des gepulsten Laserstrahls (83) zu richten, um die ringförmige Region (19) in der Hornhaut mit einer vorbestimmten Tiefe von einer vorderen Hornhautfläche (15) nach der seitlichen Schnittführung einzuschneiden,
den Fokus des gepulsten Laserstrahls zu richten, um einen ersten seitlichen Schnitt (21) in die Hornhaut nach der seitlichen Schnittführung vorzunehmen, wobei der erste seitliche Schnitt einen ersten spitzen Winkel zur ringförmigen Region (19) bildet und von einem äußeren Umfang der ringförmigen Region (19) zu einer von einer hinteren Hornhautoberfläche (27) und der vorderen Hornhautoberfläche (15) verläuft, und
den Fokus des gepulsten Laserstrahls (83) zu richten, um einen zweiten seitlichen (23) Schnitt in die Empfänger-Hornhaut nach der seitlichen Schnittführung vorzunehmen, wobei der zweite seitliche Schnitt einen zweiten spitzen Winkel zur ringförmigen Region (19) bildet und von einem inneren Umfang der ringförmigen Region (19) zur anderen der hinteren Hornhautoberfläche (27) und der vorderen Hornhautoberfläche (15) verläuft.

2. System nach Anspruch 1, ferner umfassend:
eine erste Kontaktlinse (13), die dafür geeignet ist, auf eine vordere Spender-Hornhautoberfläche (15) gelegt zu werden, wobei die erste Kontaktlinse dafür geeignet ist, die vordere Spender-Hornhautoberfläche (15) an eine Form der ersten Kontaktlinse anzupassen; und
eine zweite Kontaktlinse, die dafür geeignet ist, auf eine vordere Empfänger-Hornhautoberfläche gelegt zu werden, wobei die zweite Kontaktlinse dafür geeignet ist, die vordere Empfänger-Hornhautoberfläche an eine Form der zweiten Kontaktlinse anzupassen, wobei die erste und die zweite Kontaktlinse identische Krümmungen der hinteren Oberflächen aufweisen.

3. System nach Anspruch 2, wobei die erste Kontaktlinse (13) dafür eingerichtet ist, die vordere Spender-Hornhautoberfläche zu applanieren.

4. Verfahren zur Resektion von Spender-Hornhautgewebe zum Transplantieren von einer Spender-Hornhaut (11) auf eine Empfänger-Hornhaut, um ein Hornhautimplantat in der Empfänger-Hornhaut bereitzustellen, wobei das Verfahren umfasst:
Bestimmen einer Schnitttiefe von einer vorderen Hornhautoberfläche (15);
Durchführen eines ringförmigen Schnitts (19) mit der Schnitttiefe in die Spender-Hornhaut (11);
Durchführen eines ersten seitlichen Schnitts (21) in die Spender-Hornhaut (11), wobei der erste seitliche Schnitt einen ersten spitzen Winkel mit dem ringförmigen Schnitt (19) bildet und von einem äußeren Umfang des ringförmigen Schnitts (19) zu einer von der vorderen Hornhautoberflache (15) und einer hinteren Hornhautoberfläche (27) verläuft; und
Durchführen eines zweiten seitlichen Schnitts (23) in die Spender-Hornhaut (11), wobei der zweite seitliche Schnitt einen zweiten spitzen Winkel mit dem ringförmigen Schnitt bildet und von einem inneren Umfang des ringförmigen Schnitts (19) zur anderen von der vorderen Hornhautoberfläche (15) und der hinteren Hornhautoberfläche (27) verläuft, wobei durch eine Kombination der Schnitte in die Hornhaut Hornhautgewebe reseziert wird.

5. Verfahren nach Anspruch 4, wobei der erste seitliche Schnitt zur vorderen Hornhautoberfläche verläuft und der zweite seitliche Schnitt zur hinteren Hornhautoberfläche verläuft.

6. Verfahren nach Anspruch 4, wobei das Verfahren ferner umfasst:
Bestimmen einer weiteren Schnitttiefe von einer vorderen Hornhautoberfläche (15);
Durchführen eines Resektionsschnitts (51) mit einer ersten der Schnitttiefen in der Spender-Hornhaut (11) ;
Durchführen des ringförmigen Schnitts (19) mit einer zweiten der Schnitttiefen in die Spender-Hornhaut (11), wobei die zweite Schnitttiefe geringer ist als die erste Schnitttiefe;
Durchführen des ersten seitlichen Schnitts (21) derart, dass der erste seitliche Schnitt beim Schnitt der Spender-Hornhaut vom äußeren Umfang des ringförmigen Schnitts (19) zu einem von der vorderen Hornhautoberfläche (15) und dem Resektionsschnitt (51) verläuft, statt zu einer von der vorderen Hornhautoberfläche und der hinteren Hornhautoberfläche; und
Durchführen des zweiten seitlichen Schnitts (23) in die Spender-Hornhaut derart, dass der zweite seitliche Schnitt vom inneren Umfang des ringförmigen Schnitts (19) zum anderen von der vorderen Hornhautoberfläche (15) und dem Resektionsschnitt (51) verläuft, statt zur anderen von der vorderen Hornhautoberfläche und der hinteren Hornhautoberfläche, wobei durch eine Kombination der Schnitte in die Hornhaut Hornhautgewebe aus der Hornhaut reseziert wird.

7. Verfahren nach Anspruch 4, wobei das Verfahren ferner umfasst:
Bestimmen einer weiteren Schnitttiefe von der vorderen Hornhautoberfläche (15);
Durchführen eines Resektionsschnitts (63) mit einer ersten der Schnitttiefen in die Spender-Hornhaut (11) ;
Durchführen des ringförmigen Schnitts (19) mit einer zweiten der Schnitttiefen in die Spender-Hornhaut (11), wobei die zweite Schnitttiefe größer ist als die erste Schnitttiefe;
Durchführen des ersten seitlichen Schnitts (21) in die Spender-Hornhaut derart, dass der erste seitliche Schnitt vom äußeren Umfang des ringförmigen Schnitts (19) zu einer von einer hinteren Hornhautoberfläche (27) und dem Resektionsschnitt (63) verläuft, statt zu einer von der vorderen Hornhautoberfläche und der hinteren Hornhautoberfläche; und
Durchführen des zweiten seitlichen Schnitts (23) in die Spender-Hornhaut derart, dass der zweite seitliche Schnitt vom inneren Umfang des ringförmigen Schnitts (19) zum anderen von der hinteren Hornhautoberfläche (27) und dem Resektionsschnitt (63) verläuft, statt zur anderen von der vorderen Hornhautoberfläche und der hinteren Hornhautoberfläche, wobei durch eine Kombination der Schnitte in die Hornhaut Hornhautgewebe aus der Hornhaut reseziert wird.

## Revendications

1. Système de résection de tissu cornéen d'un donneur et de tissu cornéen d'un receveur avant transplantation du tissu cornéen du donneur comme implant cornéen, le système comprenant :
un laser chirurgical (81) adapté pour émettre un faisceau laser pulsé (83) ;
un ensemble de focalisation (85) adapté pour focaliser le faisceau laser pulsé dans une cornée ;
une interface (93) adaptée pour fournir une pluralité de motifs d'incision pour sélection d'un motif de coupe latérale qui comporte une région annulaire ; et
un contrôleur (91) en communication avec l'interface pour recevoir le motif de coupe latérale, le contrôleur étant adapté pour déplacer un foyer du faisceau laser pulsé à l'intérieur de la cornée en utilisant l'ensemble de focalisation,
orienter le foyer du faisceau laser pulsé (83) pour inciser la région annulaire (19) dans la cornée à une profondeur prédéterminée depuis une surface cornéenne antérieure (15) en fonction du motif de coupe latérale,
orienter le foyer du faisceau laser pulsé pour pratiquer une première incision latérale (21) dans la cornée en fonction du motif de coupe latérale, la première coupe latérale formant un premier angle aigu avec la région annulaire (19) et partant d'une périphérie extérieure de la région annulaire (19) vers une surface parmi une surface cornéenne postérieure (27) et la surface cornéenne antérieure (15), et
orienter le foyer du faisceau laser pulsé (83) pour pratiquer une deuxième incision latérale (23) dans la cornée du receveur en fonction du motif de coupe latérale, la deuxième coupe latérale formant un deuxième angle aigu avec la région annulaire (19) et partant d'une périphérie intérieure de la région annulaire (19) vers l'autre surface parmi la surface cornéenne postérieure (27) et la surface cornéenne antérieure (15).

2. Système de la revendication 1 comprenant en outre :
une première lentille de contact (13) adaptée pour être placée contre une surface cornéenne antérieure du donneur (15), la première lentille de contact étant adaptée pour que la surface cornéenne antérieure du donneur (15) épouse une forme de la première lentille de contact ; et
une deuxième lentille de contact adaptée pour être placée contre une surface cornéenne antérieure du receveur, la deuxième lentille de contact étant adaptée pour que la surface cornéenne antérieure du receveur épouse une forme de la deuxième lentille de contact, les première et deuxième lentilles de contact ayant des courbures de surface postérieure identiques.

3. Système de la revendication 2, dans lequel la première lentille de contact (13) est configurée pour aplanir la surface cornéenne antérieure du donneur.

4. Procédé de résection de tissu cornéen d'un donneur à transplanter d'une cornée du donneur (11) à une cornée d'un receveur pour introduire un greffon cornéen dans la cornée du receveur, le procédé comprenant les étapes suivantes :
déterminer une profondeur d'incision depuis une surface cornéenne antérieure (15) ;
pratiquer une incision annulaire (19) à la profondeur d'incision dans la cornée du donneur (11) ;
pratiquer une première incision latérale (21) dans la cornée du donneur (11), la première incision latérale formant un premier angle aigu avec l'incision annulaire (19) et partant d'une périphérie extérieure de l'incision annulaire (19) vers une surface parmi la surface cornéenne antérieure (15) et une surface cornéenne postérieure (27) ; et
pratiquer une deuxième incision latérale (23) dans la cornée du donneur (11), la deuxième incision latérale formant un deuxième angle aigu avec l'incision annulaire et partant d'une périphérie intérieure de l'incision annulaire (19) vers l'autre surface parmi la surface cornéenne antérieure (15) et la surface cornéenne postérieure (27), une combinaison des incisions dans la cornée réséquant du tissu cornéen.

5. Procédé selon la revendication 4, dans lequel la première coupe latérale va jusqu'à la surface cornéenne antérieure et la deuxième coupe latérale va jusqu'à la surface cornéenne postérieure.

6. Procédé selon la revendication 4, le procédé comprenant en outre les étapes suivantes :
déterminer une autre profondeur d'incision depuis une surface cornéenne antérieure (15) ;
pratiquer une incision de résection (51) à une première des profondeurs d'incision dans la cornée du donneur (11) ;
pratiquer l'incision annulaire (19) à une deuxième des profondeurs d'incision dans la cornée du donneur (11), la deuxième profondeur d'incision étant inférieure à la première profondeur d'incision ;
pratiquer la première incision latérale (21) de telle sorte que la première coupe latérale dans l'incision de la cornée du donneur aille de la périphérie extérieure de l'incision annulaire (19) jusqu'à une surface parmi la surface cornéenne antérieure (15) et l'incision de résection (51), plutôt que jusqu'à une surface parmi la surface cornéenne antérieure et la surface cornéenne postérieure ; et
pratiquer la deuxième incision latérale (23) dans la cornée du donneur de telle sorte que la deuxième incision latérale aille de la périphérie intérieure de l'incision annulaire (19) jusqu'à l'autre surface parmi la surface cornéenne antérieure (15) et l'incision de résection (51), plutôt que jusqu'à l'autre surface parmi la surface cornéenne antérieure et la surface cornéenne postérieure, une combinaison des incisions dans la cornée réséquant du tissu cornéen de la cornée.

7. Procédé selon la revendication 4, le procédé comprenant en outre les étapes suivantes :
déterminer une autre profondeur d'incision depuis une surface cornéenne antérieure (15) ;
pratiquer une incision de résection (63) à une première des profondeurs d'incision dans la cornée du donneur (11) ;
pratiquer l'incision annulaire (19) à une deuxième des profondeurs d'incision dans la cornée du donneur (11), la deuxième profondeur d'incision étant supérieure à la première profondeur d'incision ;
pratiquer la première incision latérale (21) dans la cornée du donneur de telle sorte que la première incision latérale aille de la périphérie extérieure de l'incision annulaire (19) jusqu'à une surface parmi une surface cornéenne postérieure (27) et l'incision de résection (63), plutôt que jusqu'à une surface parmi la surface cornéenne antérieure et la surface cornéenne postérieure ; et
pratiquer la deuxième incision latérale (23) dans la cornée du donneur de telle sorte que la deuxième incision latérale aille de la périphérie intérieure de l'incision annulaire (19) jusqu'à l'autre surface parmi la surface cornéenne postérieure (27) et l'incision de résection (63), plutôt que jusqu'à l'autre surface parmi la surface cornéenne antérieure et la surface cornéenne postérieure, une combinaison des incisions réséquant du tissu cornéen de la cornée.
